(19) **Europäisches Patentamt / European Patent Office / Office européen des brevets**

(11) **EP 1 777 518 A1**

(12) **EUROPEAN PATENT APPLICATION**
published in accordance with Art. 158(3) EPC

(43) Date of publication:
**25.04.2007 Bulletin 2007/17**

(21) Application number: **05770344.9**

(22) Date of filing: **10.08.2005**

(51) Int Cl.:
**G01N 33/50** (2006.01)    **G01N 23/223** (2006.01)
**G01N 33/483** (2006.01)

(86) International application number:
**PCT/JP2005/014627**

(87) International publication number:
**WO 2006/016597 (16.02.2006 Gazette 2006/07)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **13.08.2004 JP 2004236197**

(71) Applicant: **Chikawa, Junichi**
**Komae-city, Tokyo 201-0001 (JP)**

(72) Inventor: **Chikawa, Junichi**
**Komae-city, Tokyo 201-0001 (JP)**

(74) Representative: **Schickedanz, Willi**
**Langener Strasse 68**
**D-63073 Offenbach (DE)**

(54) **METHOD OF EVALUATING PHYSICAL CONDITIONS USING HEAD HAIR OR BODY HAIR**

(57)     It is intended to provide a method of improving dietary life or screening cancer which comprises measuring the content of an element in head hair or body hair, paying attention to the fact that the content of the element remains constant in healthy humans (i.e., homeostasis) and thus evaluating the physical conditions based on changes in the homeostasis. Namely, a method of evaluating the physical conditions whereby the physical conditions can be evaluated by measuring the content of an element in an arbitrary part of head hair or body hair. Although one's physical conditions change everyday, these changes are scarcely self-noticeable. Therefore, it is required to objectively indicate such changes in the physical conditions in numerical terms. Because of being solid, head hair or body hair can be easily handled. Thus, they are most suitable for analyzing the content of an element and enable large-scaled diagnosis at fixed intervals. This method of evaluating the physical conditions using head hair or body hair, whereby the content of an element at present can be measured by analyzing the hair root while its content of the past can be measured by analyzing at an arbitrary part, is highly effective in evaluating the physical conditions.

**FIG. 2**

EP 1 777 518 A1

## Description

## Technical Field

The present invention relates to a method for evaluating physical conditions which diagnoses the health condition of a subject by measuring the content of a specific element, and more relates to the method for evaluating physical conditions which diagnoses the health condition of the subject by measuring the concentration of the specific element for the subject and comparing it with the concentration of healthy person.

## Background Art

Modern society holds various problems and the problem for health is also one of them. At the present age when scientific technology progressed, we have received the benefit of new substances, but we are also exposed to the attack of harmful reaction of the new substances. Since the number of affection regarding allergic disease being a typical modern disease rapidly increases, it is inferred that there is a limitation also in maintaining health. Therefore, in order to live in modern society healthfully, it becomes important to grasp always our health condition and to know the abnormalities of health condition early.

As the simple methods for evaluating the physical conditions from the past, it has been generally performed to evaluate from complexion of the subject or from the condition of one part of body such as a skin and a pawl. To be sure, the condition of the complexion or the skin shows the quality of current condition. However, by these evaluation approaches, the extent of quality of the physical conditions cannot be judged objectively, and the time when the abnormal condition had occurred cannot be known, too. Therefore, it is required to present an evaluation method which can judge the change of physical conditions objectively and moreover can measure the occurrence times of abnormalities not only at present but also at the past.

Although the physical conditions are changing daily, it is rare to notice its change, so it is desired to express the change of the physical conditions by quantifying objectively. In order to quantify the change of the physical conditions objectively, the extracted blood etc. must be analyzed. The result of analysis shows the contents of elements that constitutes the blood at this time, and cannot show the variation based on time progress. Therefore, even if it becomes clear that the result of inspection is unusual, the outbreak time or elapsed time of the abnormality cannot be grasped correctly. On the other hand, blood has the work that maintains homeostasis, so that the abnormalities are not shown in many cases even when it is unhealthy. Moreover, pain, time and great costs are needed for extracting and analyzing the blood.

The body consists of 29 kinds of elements, and since its most part is water, hydrogen occupies more than one half (60.3%), oxygen 25%, carbon 10.5%, nitrogen 2.4%, so that these four kinds of element occupy 98.2%. The trace elements other than said elements are inorganic, and they are generally called mineral such as iron, copper, calcium and magnesium. Therefore, if these trace elements, especially changes in trace elements carrying out important work in life activities are analyzed, the change of physical conditions can be known promptly and it will become possible to adopt the effective countermeasures.

Especially, although calcium is an important element that constitutes the frame of body, it is carrying out other important work besides this. For example, calcium element is contained in biomembrane, so that it stabilizes the structure of this membrane, and the matter permeability of said biomembrane is maintained. Moreover, calcium element is a neurotransmitter relating to stimulus and contraction of muscle, and also relates to stimulus and secretion function of exocrine gland and endocrine gland.

In particular, when calcium runs short, bony calcium begins to dissolve into blood, and it is going to keep the calcium concentration in blood constant, so that although calcium being short, the intracellular calcium concentration is raised up, and vicious circle of causing the function fall of a cell begins. In general, this is called "calcium paradox".

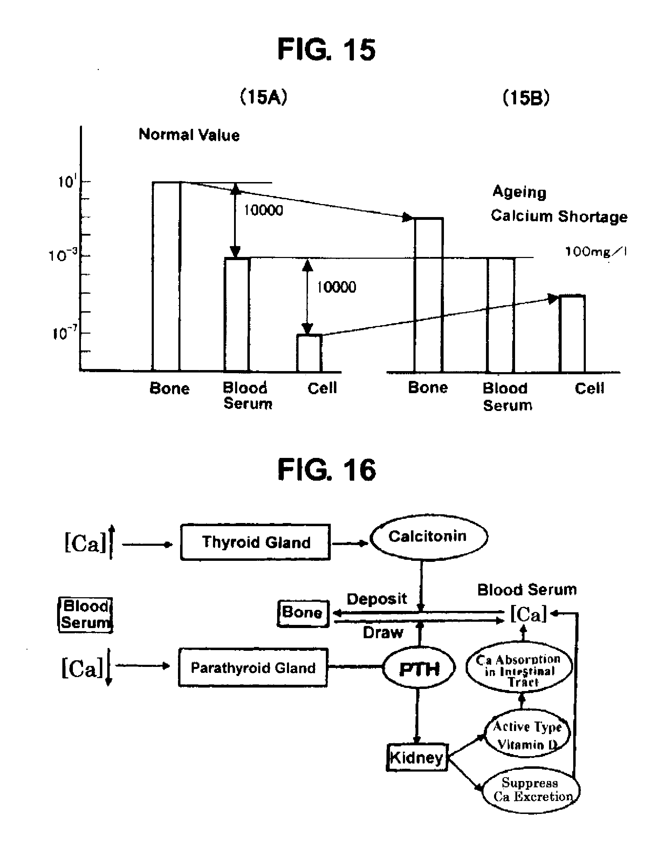

Fig. 15 is an outlined explanatory diagram of "calcium paradox". The axis of abscissa expresses each item of bone, blood serum and inside of cell, and the axis of ordinate expresses the molar quantity. Calcium not only becomes the ingredient of the frame of body, but is carrying out very important roles for the body. One of them is a role of signal transmitter in nerve. Therefore, the calcium concentration [Ca] in blood balances with bony calcium concentration, and is strictly controlled to be constant value (0.1 g in blood 1 L). Hereafter, the unit of liter is written by L.

Fig. (15A) is a conceptual diagram showing the calcium concentration of normal state contained in bone, blood and inside of cell. The bony calcium concentration [Ca] is 10000 times [Ca] in blood serum, and the intracellular calcium concentration is 1/10000 times the [Ca] in blood serum; it is seen that calcium hardly exists in cell. Therefore, cell can quickly react to concentration change of calcium being the signal transmitter, and can be normally work based on this reactivity.

Fig. (15B) is a conceptual diagram showing the calcium concentration in aging or calcium shortage condition. When calcium in blood runs short, calcium begins to dissolve into blood from bone being the huge storehouse of calcium, the calcium concentration in blood tends to be constant, and it is going to maintain the neural transmission operation normally. At the same time, cell becomes full of calcium and calcium concentration becomes high, so that the function of cell deteriorates because of worsening of signal transmission due to calcium, then there are caused various illnesses such as not only osteoporosis but also immunity disease, diabetes, hypertension, malignant tumor, and arteriosclerosis. As described above, in spite of calcium shortage, the phenomenon in which intracellular calcium concentration rises is apparently a contradictory phenomenon, so it is called "calcium paradox."

Fig. 16 is an outlined explanatory diagram showing the system by which the calcium concentration [Ca] in blood is controlled to be the constant value. When [Ca] in blood increases, calcitonin is secreted from the thyroid gland, and calcium in blood (blood serum) is deposited in bone. On the other hand, when [Ca] in blood decreases, parathyroid hormone (PTH:Parathyroid Hormone) is secreted from the parathyroid gland, and calcium is extracted from bone into blood. PTH controls the excretion of calcium to urine by acting on kidney, and promotes the absorption of calcium from intestines by activating vitamin D. Thus, PTH increases [Ca] in blood, at the same time, makes overflow of calcium in the cells of body, and becomes the cause of "calcium paradox".

Although "calcium paradox" is apparently the contradictory phenomenon, when it is considered well, it is found that calcium plays the very important roles in life activity of human body. Therefore, if it can be diagnosed whether calcium is short by a simple method, changes of physical conditions can be predicted, and it can be used for judging of a sick omen or a sick progress. If the concentration of PTH in blood is measured, it can be diagnosed whether calcium is short, but the concentration of PTH changes with a one-day cycle, and a PTH molecule is broken easily. Moreover, since its fragments also function, measurement is not easy. As it is necessary to extract and inspect blood for that purpose, it has not been used widely until now because of time and cost.

It has been considered so far that there is individual difference in the content of an element contained in hair and the content of the element varies depending on place and time of taking hair even for the same person's hair. In order to obtain the content of the element in such hair, hairs of several grams were taken, and the content of each element was analyzed and obtained by atomic absorption analysis etc. However, this method measures the average value of the content of the element in hair, and the measured value is the average value for at least one month. And since the content of an element changes more quickly, there is the disadvantage that the value at the measurement time is not acquired.

Also, X-ray fluorescence analysis is desirable as an analytical method of the content of each element in hair. This X-ray fluorescence analysis is an analysis method, in which electron beam or X-ray beam irradiates on a sample, the generated fluorescent X-rays having the wavelength peculiar to the element is detected, and the kind and amount of the elements contained in the sample are measured. However, since the wavelength of the irradiated X-rays in the conventional analysis method spreads widely, it laps with the wavelength of the generated fluorescent X-rays, so that the high sensitivity is not obtained because of the noise, so exact analysis has been difficult.

Furthermore, since the fluorescent X-rays intensity increases proportionally to the mass of the element in the part to which the excitation beam is irradiated, the measurement data change with various hairs such as thick hair, thin hair and curled hair. Therefore, although the absolute measurement of the concentration, namely how much mg of the element is contained in 1 g of hair, is required, it is difficult to measure the mass of the irradiated part correctly for each sample.

As a result of studying deeply for above-mentioned problem, the present inventor paid his attention especially to the hair root of head hair or body hair on the basis of assumption that "calcium paradox" due to shortage of calcium occurs similarly in every cell in the living body. Head hair or body hair is supported by hair root, and the nutrient is supplied from hair matrix cells wrapping the hair root. Therefore, when measuring the amount of calcium in this hair root, it is surmised that the shortage of calcium could be grasped and the research was repeated wholeheartedly.

Consequently, the present inventor proposed the diagnostic method of physical conditions based on head hair or body hair by Japanese Patent Laid-Open No. 2004-45133 (patent reference 1), and made an oral announcement of the contents of said patent reference 1 in English in the international congress of Ritsumeikan University for two days of January 12 and 13 of 2004. This diagnostic method is characterized in that many trace elements contained in hair root of head hair or body hair are detected and then the content ratios among elements are calculated.

[Patent Reference 1] Japanese Patent Laid-Open No. 2004-45133

**Description of the Invention**

**Problems to be Solved by the Invention**

Said patent reference 1 aimed at solution of the above-mentioned problems through the steps of irradiating the synchrotron radiation X-rays to hair root of head hair or body hair, measuring contents of the elements contained in the hair root, and obtaining the content ratios of elements.

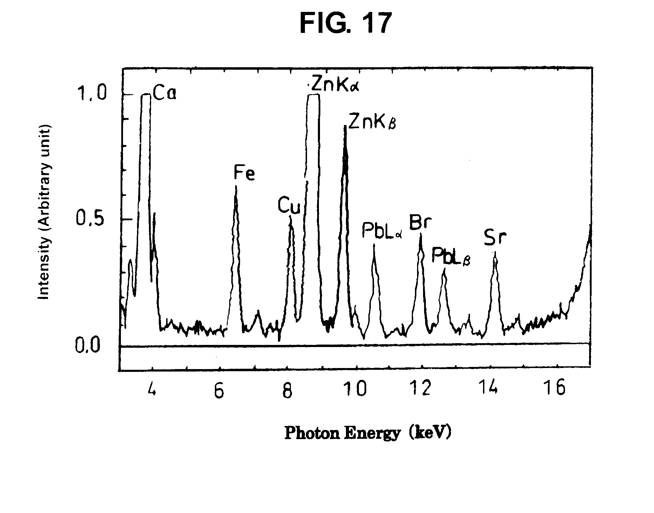

Fig. 17 is a spectrum diagram of the content of element contained in hair root in the patent reference 1. Photon Energy (keV) is expressed with an axis of abscissa, and Intensity (arbitrary unit) is expressed with an axis of ordinate. The detected elements are calcium (Ca), iron (Fe), copper (Cu), zinc (Zn), lead (Pb), bromine (Br) and strontium (Sr). The pulses generated at photon energy (wavelength) proper to an element are accumulated, and the accumulated amount is expressed as fluorescent X-ray intensity for proper photon energy of the element. Since the fluorescent X- ray intensity is proportional to the content of the element, the ratio of said intensity to the fluorescent X-ray intensity of a standard element of the same person is obtained, and this ratio expresses the content ratio of said element.

In said patent reference 1, although the content ratios of other elements are obtained by adopting the iron (Fe) contained in the hair root of head hair or body hair as a standard, it cannot be concluded that the content of iron in the hair root is always constant, and its contents are different for individuals. Therefore, the diagnostic method by change of the content ratio of element obtained by this approach lacks strict preciseness, and the diagnosis by comparison with the content ratio of the element for other healthy person also lacks accuracy.

Moreover, the patent reference 1 is limited to hair root of head hair or body hair, and is characterized by measuring the content of element in the hair root and diagnosing the physical conditions. Since the hair root is covered with hair matrix cells, it is thought that the change of physical conditions is reflected clearly. However, the content of element contained in the hair root is the content at present time, and is not the content at past time. Therefore, as the hair root analysis of head hair or body hair described in the patent reference 1 can grasp only the variation of the content of element at present time, the exact diagnosis of physical conditions cannot be performed.

As a result of sharp and zealous research by the present inventor, it is proposed that the concentration can be correctly derived as a relative value by logarithmic expression of the content of element instead of the content ratio of element, and it is concluded that measurement by analyzing an arbitrary part of head hair or body hair can give the content of element at that time point. Consequently, it is discovered that the important element such as calcium, iron, copper, zinc and selenium etc. contained in hair is controlled to have the constant concentration and has the universal concentration for healthy persons, so that the present invention is completed.

Therefore, the purpose of the present invention is to provide a method to know the concentration homeostasis in hair for healthy persons and to make improvement of dietary habits and cancer screening by evaluating physical conditions as deviations from said homeostasis.

**Means for Solving the Problem**

The present invention is done to solve said problems, and the first form of the present invention is a method for evaluating physical conditions by measuring content of an element contained in an arbitrary point of head hair or body hair.

The second form of the present invention is the method for evaluating physical conditions, wherein said contents of the element contained in two or more points of said head hair or body hair are measured, and a time change of said contents of the element is derived by comparing said contents of the element.

The third form of the present invention is the method for evaluating physical conditions, wherein said evaluation of the physical conditions is performed by the use of said one or more head hairs or body hairs taken periodically.

The fourth form of the present invention is the method for evaluating physical conditions, wherein said evaluation of the physical conditions is performed by comparing said contents of the element in said head hair or body hair with the content of the element contained in other specimens aside from hair taken from the same person.

The fifth form of the present invention is the method for evaluating physical conditions, wherein said content of the element contained in said head hair or body hair of a subject is compared with content of the element contained in head hair or body hair of a healthy person, and said physical conditions is evaluated to be abnormal when said content of the element of said subject is larger or smaller than that of said healthy person.

The sixth form of the present invention is the method for evaluating physical conditions, wherein said content of the element is a concentration of the element that is calculated from said content of the element at said measurement point of said head hair or body hair.

The seventh form of the present invention is the method for evaluating physical conditions, wherein an excitation beam is irradiated to said measurement point of said head hair or body hair, fluorescent X-rays emitted from said measurement point are measured, and said concentration of the element is derived from said fluorescent X-ray intensity.

The eighth form of the present invention is the method for evaluating physical conditions, wherein said fluorescent X-ray intensity (P) is expressed by the decimal logarithm (logP) and said concentration of the element is given by the value (logP-logS) obtained by deducting the background value (logS) independent of the kind of element.

The ninth form of the present invention is the method for evaluating physical conditions, wherein said concentration of the element for said subject is written as subject concentration (logP-logS), said concentration of the element for said healthy person is written as healthy person concentration (logP-logS)st, the normalized concentration M is defined by the formula logM = (logP - logS)/(logP - logS)st, and said evaluation for the physical conditions is performed by said normalized concentration M.

The tenth form of the present invention is the method for evaluating physical conditions, wherein said element is a trace element such as Ca, Fe, Cu, Zn, Se, Sr and Rb.

The eleventh form of the present invention is the method for evaluating physical conditions, wherein said excitation beam is synchrotron radiation.

The twelfth form of the present invention is the method for evaluating physical conditions, wherein said evaluation is a diagnosis of breast cancer.

The 13th form of the present invention is the method for evaluating physical conditions, wherein said evaluation is performed together with medical detailed examination.

**Effect of the Invention**

According to the first form of the present invention, the content of the element contained in an arbitrary part of head hair or body hair can be measured, so that the physical conditions can be evaluated. In the patent reference1, the analysis was performed by limitation to hair root of head hair or body hair. Therefore, since the content of element in the hair root is the content of element at present time, only the change of present physical conditions becomes clear from the result of evaluation. The present invention was completed through paying attention to that the content of element at present time can be measured by analyzing the hair root of head hair or body hair and that analyzing an arbitrary part of the hair can give the content of element at that time point. Since head hair or body hair is growing from the hair root, the arbitrary part reflects the content of element of hair root at past clearly. Therefore, if the arbitrary part of hair is analyzed, the content of element at that time can be measured, and if the hair root is analyzed, the present content of element can be measured, so that it is possible to grasp the change of physical conditions dating back to the past. As a result, since the method for evaluating physical conditions of the present invention can grasp the time to make change of the physical conditions, it can be used for a diagnosis of a sick omen or a sick progress. Moreover, since head hair or body hair is a solid, it is easy to handle, it is optimal for analyzing the content of element, and it is easy to perform a lot of diagnosis periodically. Therefore, the evaluation of physical conditions by head hair or body hair is the very effective diagnostic approach.

According to the second form of the present invention, the contents of the element contained in two or more points of head hair or body hair are measured, and a time change of said contents of the element can be derived by comparing said contents of the element. Therefore, the record that comes back in the past by analysis of one hair, namely the hysteresis information on the content of element can be investigated simply. Analysis of the hair root gives the present information, and the information on past can be analyzed along hair shaft to the hair tip. Since 1 cm of hair corresponds to about one month, the tip point at 12cm apart from the hair root gives the information of one year ago. Consequently, even if there is no abnormality in the content of element at present time, it not only can be investigated whether there were certain abnormalities at the time of the past, but the time elapsing from an abnormal occurrence and the time variation of the content of element can be grasped, so that it becomes an important judgment standard when diagnosing a sick omen and a sick progress.

According to the third form of the present invention, since the evaluation of the physical conditions can be performed by using of one or more head hairs or body hairs extracted periodically, it is possible to investigate not only the hysteresis information of the content of element contained in hair at present time but also the hysteresis information coming back to the past. The hysteresis information on the content of element contained in one hair depends on its length. Long hair has the information of a long period and short hair has the information of a short period. Therefore, if hairs are periodically extracted according to their lengths, the change of the physical conditions over a long period can be investigated, so that the time to produce abnormalities in the physical conditions can be grasped correctly. Consequently, the cause of the abnormalities in physical conditions can be traced and it can be used for the daily health care and sick protection. Moreover, if the time interval for taking hair is shortened, it is possible to avoid the change of the content of element in hair due to chemicals contained in hair cosmetics etc., so that exact variation can be measured. Moreover, the trace element is contained in hair dye and moves into hair. For example, since calcium has the tendency to move into hair and to form pair atoms with calcium in hair, the effect of hair dye can be deducted.

According to the fourth form of the present invention, the evaluation of the physical conditions is performed by comparing the contents of the element of said head hair or body hair with the content of the element contained in other specimen besides hair from the same person. By using of the method for evaluating physical conditions of the present invention, it gets possible to grasp not only the deviation from the healthy value of the content of element contained in hair but the relation with the content of element contained in other specimens besides hair such as blood and tissue pieces of the same person, so that more exact analysis can be performed. Therefore, as to abnormalities of the content of element that cannot be judged only by analysis of the content of specific element contained in hair, an exact diagnosis can be attained by analyzing the relation with the content of element contained in other specimens of the same person.

According to the fifth form of the present invention, when the measured content of the element of the subject is larger or smaller than the content of element of healthy person, the physical conditions are evaluated to be abnormal. The present inventor discovered that the content of an element in the hair of healthy persons is controlled to be constant, and has a value to be common for healthy persons. By proposing to detect the change of physical conditions promptly by the deviations from this homeostasis, the present invention was completed. Therefore, since the contents of the element of each person are almost the same during a healthy period, it can be found that there is certain abnormality when large or small as compared with this value. It can be used for sick prophylaxis by investigating the cause of this abnormality.

According to the sixth form of the present invention, since the measured content of element is the concentration of element in the measured part of head hair or body hair, it can be compared with the content of element contained in other specimens from the same person and the content of element of healthy person, and proportionality between them can be investigated. The thickness of the measured head hair or body hair varies from sample to sample, resulting in different masses or molar quantities as a content of element even for healthy person. Then, in this form, the concentration of element is adopted as a content of element, and for example, if the element mass per unit mass of hair is taken, they must be mostly in agreement among healthy persons. Therefore, by comparing the concentration of element of the subject with healthy concentration, an exact evaluation of physical conditions becomes possible. It is possible to evaluate precisely the physical conditions from the relation of the concentration of element of head hair or body hair with the concentration of element contained in other specimens from the same person besides head hair or body hair and the concentration of element of healthy person.

According to the seventh form of the present invention, since the concentration of element can be derived by measuring of fluorescent X-rays emitted from the measurement part of head hair or body hair, the exact concentration of element can be obtained. In X-ray fluorescence analysis, although it is desired to analyze the samples for comparison under the conditions as same as possible, analysis can be performed freely from any other conditions. Therefore, the obtained analytical results have a very high precision, and can be sufficiently used as the standard for comparison. Moreover, since it can analyze independently of analyst's capability and the conditions in the analysis, there is an advantage to obtain exact distribution data for elemental concentration.

According to the eighth form of the present invention, the fluorescent X-ray intensity (P) is expressed by the decimal logarithm (logP) and said concentration of the element is given by the value (logP-logS) obtained by deducting the background value (logS) independent of the kind of element from said decimal logarithm value (logP). Since it is logP-logS=log (P/S), this P/S gives the concentration value of an element, so that if said fluorescent X-ray intensity (P) is expressed by logarithm, the concentration of an element can be derived easily. Fluorescent X-ray intensity (P) changes according to thickness of hair, and if the intensity per unit mass is set to N, it will become P=SN, where S is a proportional constant which varies according to the thickness of hair. Therefore, in expression of decimal logarithm, it is as follows.

$$P=SN \quad \therefore \ logP=logSN=logN+logS$$

logS is the background without wavelength dependency in fluorescent X-ray intensity. Therefore, if the fluorescent X-ray intensity (P) is expressed by the decimal logarithm (logP), and if the amount (logP-logS=logN) that reduced the background value (logs) is graphized as ordinate axis, it is found that the height of its graph shows the concentration (logN) of an element. As mentioned above, this logN or N is equivalent to the concentration of element, and an exact evaluation of physical conditions is attained with comparing said concentration of element.

According to the ninth form of the present invention, said concentration of the element for said subject is written as subject concentration (logP-logS), said concentration of the element for said healthy person is written as healthy person concentration (logP-logS)st, the normalized concentration M for said subject is defined by the formula

$$logM = (logP - logS)/(logP - logS)st,$$

and by obtaining said normalized concentration, it is possible to know how many times said subject concentration is to said healthy person concentration. Furthermore, if the normalized concentration M of other specimen (of the same person) is obtained, the relation between the concentration of element of hair and the concentration of element of other specimen becomes clear, and the exact analysis of the concentration of element can be performed. Consequently, the deviation from the constant value can be quantified and the clear and objective evaluation of physical conditions can be performed.

According to the tenth form of the present invention, trace elements important for life activity such as Ca, Fe, Cu, Zn, Se, Sr and Rb are detected, their contents are measured, and the physical conditions can be evaluated from variations of the contents of elements. Ca is not only an important element constituting the body frame, but also a very important signal transmitter of cells. It has been found that the intracellular amount of Ca increases conversely when calcium runs short. Therefore, if the variation of the amount of Ca is measured, the evaluation of physical conditions can be performed. Moreover, when metallic element such as Fe, Cu, and Zn is superfluous, liver has a function to excrete the element, so that when the function deteriorates, the contents of these elements in hair will change. Moreover, trace elements such as Se, Sr, and Rb are closely connected with regulation and homeostasis of various living body functions such as high order function of central nervous system, metabolism, immunity, and oxidation stress. Therefore, it is possible to evaluate the physical conditions by measuring the variation of contents of the elements. In particular, if the amount of Ca contained in hair etc. is measured, shortage of Ca can be known easily, and illness caused by the shortage of Ca can be prevented.

According to the eleventh form of the present invention, since the excitation beam is synchrotron radiation (X-rays), X-ray fluorescence analysis with a very high precision can be performed. Since the synchrotron radiation intensity is very high, it can be used sufficiently even if it is monochromized. Therefore, highly precise X-ray fluorescence analysis with low noise is possible, and it is the best for the method evaluating for physical conditions of the present invention. Moreover, since it has high directivity and high polarization, it is very effective in the elementary analysis of a minute sample like hair.

According to the twelfth form of the present invention, it is possible to diagnose breast cancer by the method for evaluating physical conditions of the present invention. Since the method for evaluating physical conditions of the present invention can analyze not only deviation of the content of element at present time but also deviation of the content of element at past time, it can diagnose the generation of illness by abnormalities in calcium metabolism, especially breast cancer. In addition, by measuring the time variation in the content of element (especially, calcium concentration), it can be used for diagnosis of omen and progress of the breast cancer. Furthermore, if the shortage of calcium can be recovered by the method for evaluating physical conditions of the present invention, the illness by abnormalities in calcium metabolism such as osteoporosis, hypertension, arteriosclerosis, malignant tumor and immunopathy can be prevented.

According to the 13th form of the present invention, said evaluation for physical conditions can be performed together with medical detailed examination. If abnormalities are found in the content of element contained in hair by the method for evaluating physical conditions of the present invention, much more exact evaluation for physical conditions can be performed by inspecting together with a medical detailed examination such as blood test, X-ray inspection and endoscopy. Since the method for evaluating physical conditions of the present invention can diagnose easily the change of physical conditions by only measuring the content of element contained in hair, it is possible to know the abnormalities of physical conditions still earlier by carrying out it periodically and said method can promote the prophylaxis of illness.

**Brief Description of the Drawings**

[FIG. 1] FIG. 1 is a schematic diagram of X-ray fluorescence analysis using synchrotron radiation.
[FIG. 2] FIG. 2 is a spectrum diagram of hair and serum by the X-ray fluorescence analysis.
[FIG. 3] FIG. 3 is a flow diagram of the method for evaluating physical conditions to diagnose by detecting the specific element contained in hair.
[FIG. 4] FIG. 4 is a flow diagram of the method for evaluating physical conditions to diagnose by detecting calcium element contained in hair.
[FIG. 5] FIG. 5 is a flow diagram of the method for evaluating physical conditions to diagnose by detecting the specific element contained in two or more arbitrary parts of hair.
[FIG. 6] FIG. 6 is an enlarged model diagram of one hair.
[FIG. 7] FIG. 7 is a comparison diagram of the concentration of calcium contained in hair and blood serum of eight healthy persons.
[FIG. 8] FIG. 8 is a comparison diagram of the concentration of calcium contained in hair of 37 subjects.
[FIG. 9] FIG. 9 is a comparison diagram of the concentration of iron contained in hair and blood serum of eight healthy persons.
[FIG. 10] FIG. 10 is a comparison diagram of the concentration of iron contained in hair of 37 subjects.
[FIG. 11] FIG. 11 is a comparison diagram of the concentration of copper contained in hair and blood serum of eight healthy persons.
[FIG. 12] FIG. 12 is a comparison diagram of the concentration of copper contained in hair of 37 subjects.
[FIG. 13] FIG. 13 is a comparison diagram of the concentration of calcium contained in hair of 17 breast-cancer patients.
[FIG. 14] FIG. 14 is a hysteresis curve diagram of the concentration of calcium contained from hair root to tip of hair.
[FIG. 15] FIG. 15 is an outlined explanatory diagram of calcium paradox.
[FIG. 16] FIG. 16 is an outlined explanatory diagram showing the mechanism by which the concentration of calcium [Ca] in blood is controlled to be a constant value.
[FIG. 17] FIG. 17 is a spectrum diagram of the content of element contained in hair root shown in patent reference 1.

**Description of Notations**

10    Electron Storage Ring
12    Synchrotron Radiation
14    Monochromator
16    Facility Separation Wall
18    Aluminum Foil
20    Vacuum Induction Path
22    Pinhole
24    Acrylic Holder
26    Hair
28    Semiconductor Detector

## Best Mode of Carrying Out the Invention

The embodiments of the method for evaluating physical conditions of the present invention are described in detail according to the accompanying drawings as follows.

X-ray fluorescence analysis is desirable for the elementary analysis of head hair or body hair of the present invention. Although there is X-ray fluorescence analysis such as X-ray fluorescence excited by X-rays, X-ray fluorescence excited by electron beam and X-ray fluorescence excited by ion beam, X-ray fluorescence analysis excited by synchrotron radiation is desirable in the present invention.

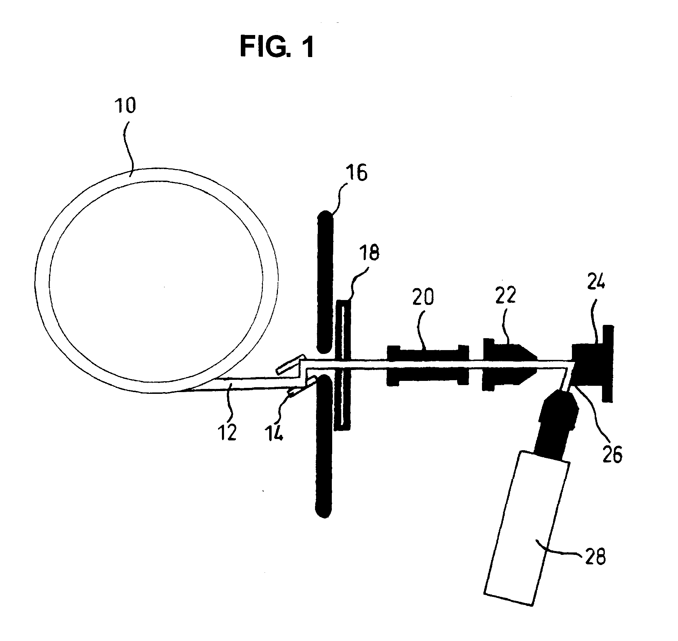

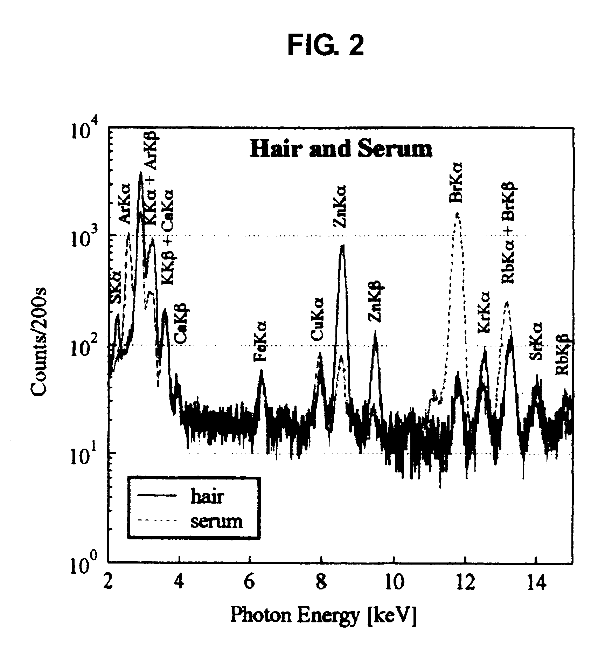

FIG. 1 is the outlined diagram of the X-ray fluorescence analysis excited by synchrotron radiation. When the electron 10 accelerated to a velocity near the velocity of light is changed its running direction, the synchrotron radiation 12 is emitted to the tangential direction of the running orbit. This synchrotron radiation 12 is monochromatized by the monochromator (wavelength monochromater) 14, so that the X-rays of 20keV are obtained. The synchrotron radiation 12 introduced from the radiation hole of the facility separation wall 16 sealed with aluminum foil 18 is focused through the vacuum induction path 20 into a narrow beam by the pinhole 22 (for example, 0.2x0.2mm), and is irradiated to the hair 26 that is a sample. The hole is opened in the center of the acrylic holder 24, and the measurement part of hair is placed in the center of the hole. The X-rays passing through the hair just go through, and it is designed not to contribute to the background of measured value. Fluorescent X-rays are emitted from the placed hair 26, and the fluorescent X-rays are detected with the semiconductor detector (SSD) 28. The detected fluorescent X-rays are resolved into energy (wavelength) spectrum by the multichannel pulse height analyzer, and the number of photons within the set-up time (for example, for 200 seconds) is measured. In general, the synchrotron radiation is the linearly polarized light in the horizontal plane, so that the intensity of scattered X-rays becomes zero theoretically in the direction perpendicular to the incident direction on this plane of polarization. Therefore, the S/N ratio is raised by measuring the fluorescent X-rays emitted in this direction. FIG. 2 is the diagram that graphed said measured number of photons.

FIG. 2 is the spectrum diagram obtained by the X-ray fluorescence analysis of head hair (Hair) and blood serum (Serum). The axis of abscissa expresses the energy (Photon Energy: keV) of fluorescent X-rays, and the axis of ordinate expresses the decimal logarithm of the number of photons for 200 seconds (Counts / 200s). Head hair and body hair are produced from the hair matrix cell in the hair follicle, and the hair follicle is surrounded by blood vessels, so that hair grows by nutrition supplied from blood (blood serum). Then, in order to investigate the relation of the concentration of element between blood serum (Serum) and head hair (Hair), hair and blood were simultaneously extracted from eight healthy subjects. The blood is applied to a centrifugal separator, and the blood serum was obtained by separating the erythrocyte. One drop of this blood serum is dropped on thin Mylar through which X-rays transmits easily, and it is dried. This Mylar means polyester film. X-ray fluorescence analysis of this dried blood serum and hair was performed, and FIG. 2 is the diagram in which their spectra are together shown so as to make their backgrounds agree with each other and this diagram shows a typical example for the case of the subject in a healthy and steady state.

In FIG. 2, the peaks of many trace elements contained in blood serum and hair are seen. The peak of zinc (Zn) in hair is higher than that in blood serum. Conversely, the peak of bromine (Br) is lower. The peaks of Ca, Fe, Cu and Sr in hair and blood serum are superimposed. In the healthy and steady state, it is found from the property of logarithm that the hair concentration of these elements is proportional to the concentration in blood serum and their proportional constants are the same. The element distributions of eight subjects in the blood serum are alike well, and the spectrum peaks of all the subjects are almost superimposed. It is found from these facts that these elements are important, the concentrations are always controlled to be constant, and they are the concentrations of elements universal for everybody. Therefore, the concentration of element in hair is kept to be constant in the healthy case, but when it becomes unhealthy, the concentration differs greatly in hair even if it is kept constant in blood serum. As a result, the shift from normal value increases in hair.

When the height of fluorescent X-ray spectrum peak of a certain element is A in thick hair and B in thin hair, the proportional relation $A = \alpha B$ holds. Here, $\alpha$ is a proportional constant, and since it is difficult to determine the value of $\alpha$ correctly, the decimal logarithm is taken. Namely, $\log A = \log \alpha B = \log B + \log \alpha$. Supposing that the concentration of said element is constant independently of thickness of hair, two peaks of logA and logB can be superimposed by shifting by $\log \alpha$.

The axis of ordinate (the number of photons) is made in a logarithmic scale, two sheets of spectra to be compared are superposed, and the axis of abscissa is arranged so as to agree with each other. Some peaks have come out on the background in the spectrum diagram. One sheet is shifted upward or downward so that their backgrounds agree well. If one peak is superimposed, the element of the peak has the same concentration (the same amount of the element in 1 g of hair). The difference in height between peaks not being superimposed gives how many times the concentration is different.

When the height of a peak is specified by logP and the background based upon X-rays scattered by the sample is specified by logS, the concentration of this element is expressed by [logP-logS] =log (P/S). This concentration of the subject [logP-logS] is called the subject concentration, and the standard value of the healthy person [logP-logS]st is called the healthy person concentration. In order to express the shift of the subject concentration from the healthy person concentration, the measured value [logP-logS] is normalized by the following formula, and the value of this normalized concentration M was plotted with logarithmic scale.

$$[logP-logS]/[logP-logS]st=logM$$

Namely, the healthy standard value is set to M=10. This approach does not carry out the absolute measurement of the content of an element, and obtains the relative measurement value with a sufficient precision, namely how many times the value is to a certain standard value such as the content of element contained in hair of a healthy person. By this method, it is possible not only to compare correctly the hair spectrum diagram of a large number of persons, but also to know the proportional relation of the concentration of each element by comparing the spectrum diagram of blood serum with that of hair.

There are always the growing hair and the falling hair in head hairs that show the hair cycle peculiar to human being, and growth period, degradation period and resting period are freely repeated for every one hair. It is said that the growth period is three to seven years. When it finishes, the hair follicle wrapping the hair root begins to prepare entering to the resting period. This is the gradation period that is said to be two to three weeks. Next, when it goes into the resting period, the hair falls out after it remained in the hair follicle for a while. After the resting period continued for three to six months, it enters in the growth period again, so that the hair follicle goes into deep part to be activated, and hair grows. Therefore, it is desirable to use the hair of the same conditions in the elementary analysis of the present invention.

In the present invention, the narrow synchrotron radiation X-ray beam (for example, 0.2 × 0.2mm) is irradiated to one head hair (body hair) (or hair bundle that arranged the hair roots), and the characteristic fluorescent X-rays generated from each element are measured, so that the homeostasis of the content of the element is found, and the healthy conditions is judged by setting said value as the standard value. Furthermore, since the head hair (body hair) grows about 10mm in length through one month, the content of one element is measured from the hair root to the tip point, so that it is possible to diagnose the sick omen and sick progress from the property of its variation. Therefore if the diagnosis of physical conditions based on hair is performed periodically, for example annually, the content of the element in the hair can be measured over several years, and the change of physical conditions can be promptly known by deviations from the constant value. Consequently, various illnesses caused with deviations of the content of element can be prevented. Next, the concrete diagnostic method is described.

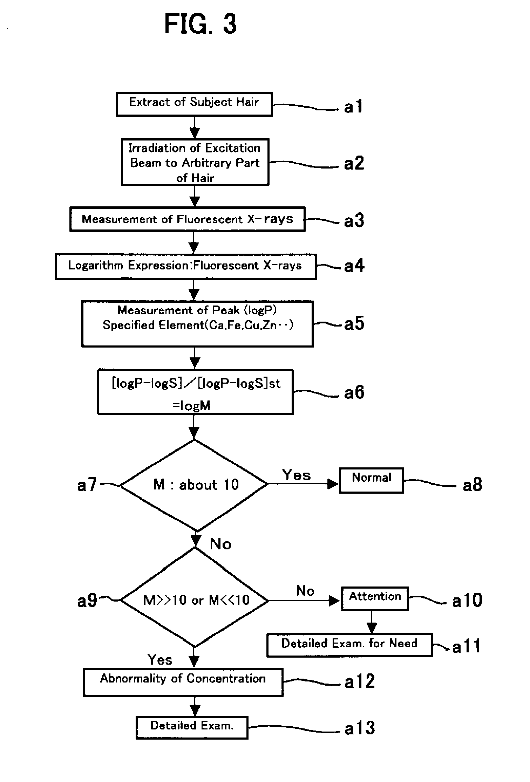

FIG. 3 is the flow diagram of the method for evaluating physical conditions to diagnose by detecting the specific element contained in hair of subject. In step a1, after a hair is extracted from the subject, the excitation beam is irradiated to the arbitrary part of the hair (a2), and the spectrum distribution of the fluorescent X-rays emitted from said arbitrary part is measured (a3). The measured fluorescent X-ray intensity is expressed by decimal logarithm (a4), and its attention is paid to the peak (logP) of the specific element (a5). The ratio of the subject concentration [logP-logS] of said specific element to the standard healthy person concentration [logP-logS]st is calculated, and this ratio is corresponding to logM (a6). In the present invention, M is called the normalized concentration, and when the subject is a healthy person, it turns out to be M = 10. It is judged whether the value of M is in the vicinity of 10 (a7), and in the case of vicinity, it is judged to be normal (a8). When it is in the case of no vicinity, it advances to the judgment of (a9) of M>>10 or M<<10. When the value of M is larger than the vicinity of 10, or when it is smaller than the vicinity of 10, it is diagnosed to be medical attention (a10), and when required, a medical detailed examination (a11) may be used together. When M is deviated greatly from 10, said medical detailed examination (a13) is needed as abnormalities in the concentration of element (a12). When said arbitrary part is the hair root, the current condition evaluation can be performed, and when it is the part distant from the hair root, it can be performed the condition evaluation at the time of the past at which the part existed at the position of hair root. Thus, the present invention is characterized in that it can measure the element distribution of the present or the past.

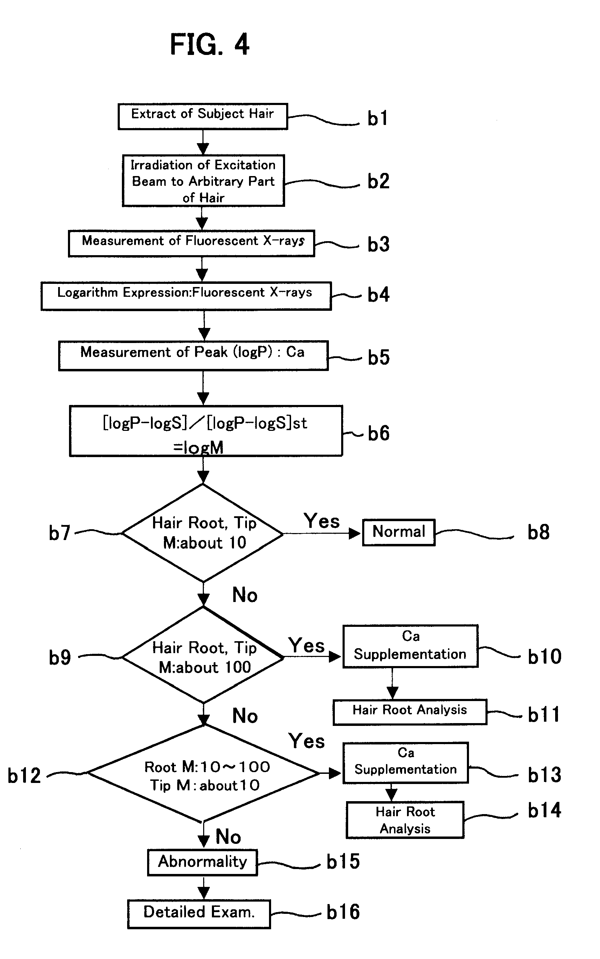

FIG. 4 is the flow diagram of the method for evaluating physical conditions to diagnose by detecting calcium element contained in hair. In step b1, after a hair is extracted from the subject, the excitation beam is irradiated to the arbitrary part of the hair (b2), and the spectrum distribution of the fluorescent X-rays emitted from said arbitrary part is measured (b3). The measured fluorescent X-ray intensity is expressed by decimal logarithm (b4), its attention is paid to the peak (logP) of calcium element (b5), so that the ratio is calculated from said concentration of calcium [logP-logS] and the concentration of calcium in healthy person [logP-logS]st, and logM is derived from said ratio (b6). Said normalized concentration M is measured for the hair root part and the tip part distant from the hair root, and whether both of M are about 10 is judged (b7). In the case of that the values of M of the hair root part and the tip part are constant in the vicinity of 10, it is judged to be normal (b8). In the case of that it is not so, it is judged whether the values of M of the hair root part and the tip part of hair are in the vicinity of 100 (b9), and if both values are about 100, the subject must take Ca agent (Ca ingredient is 900 to 1200mg) every day for about ten days (b10). After that, the hair root is newly analyzed (b11), so that the detailed examination becomes necessary in the case of that the value of M does not come back to the normal value (about 10). Furthermore, it is judged whether the value of M of the tip part is about 10 and that of the hair root is 10 to 100 (b12), and in this case, the subject must take Ca agent (Ca ingredient is 900 to 1200mg) every day for about ten days (b13). After that, the hair root is newly analyzed (b14), so that the detailed examination becomes necessary in the case of that the value of M does not come back to the normal value (about 10). When not applied also in all the above case, it is judged as concentration abnormality of element (b15), the fluctuation hysteresis of M is investigated, and the name of disease is decided by transferring to the detailed examination (b16). Said tip part is not limited to one, and in the case of a plural number, it is possible to perform the evaluation of physical conditions in more details. When the value of said M is near 100, the basis that there are the abnormalities in calcium (calcium shortage) is mentioned later. As described later, in the process that a breast cancer develops, the state that the value of M is 100 continues for a long time, and the value decreases slowly to the normal value 10 in about one year. Therefore, in the case of that the tip part shows 100 and the hair root shows the intermediate value between 10 and 100, the points between them are analyzed with interval of 1-2cm, and it is judged by investigating of the variation behavior of M.

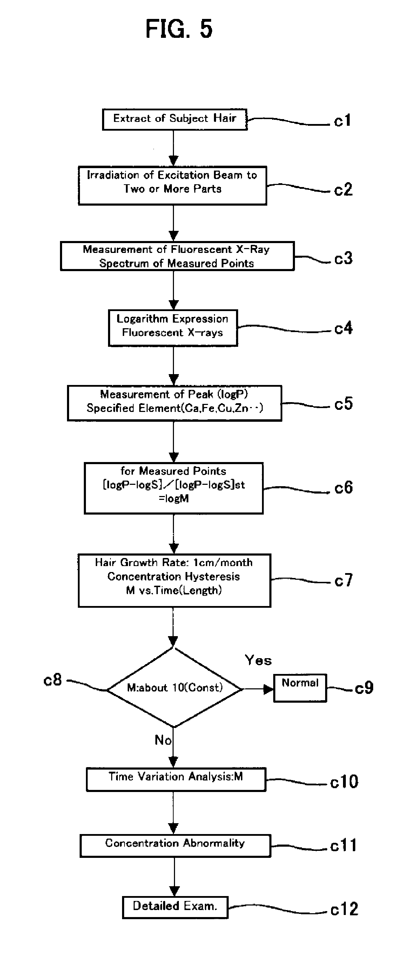

FIG. 5 is the flow diagram of the method for evaluating physical conditions to diagnose by detecting the specified element contained in two or more arbitrary parts of hair. In step c1, after a hair is extracted from the subject, the excitation beam is sequentially irradiated to the two or more arbitrary parts of the hair (c2), and the spectrum distribution of the fluorescent X-rays emitted from each of said arbitrary parts is measured (c3). Each of measured fluorescent X-ray intensities is expressed by decimal logarithm (c4) and the attention is paid to the peak (logP) of said specified element (c5). Each ratio is calculated from the concentration [logP-logS] of said specific element at each measured point and the concentration [logP-logS]st of said specific element in healthy person, and then logM of each part is obtained from said ratios (c6). The time of past when each part existed at the position of hair root is found by the distance between each part and the hair root. The more the number of measured points, the more precise the time variation of the concentration of element is measured, so that the more detailed diagnosis becomes possible. Although only current information can be analyzed by medical inspection of a blood test and others, past information can be analyzed by the hair diagnosis. Therefore, the more exact diagnosis is attained by use of hair analysis together with other inspection. There is expressed the hysteresis curve of the concentration of element (c7), in which time (length from hair root) is taken along the axis of abscissa, and M is taken along the axis of ordinate. It is judged whether the value of M is to be constant near 10 (c8), and in the constant case, it is diagnosed that the subject is normal from the past to the present (c9). In the case that there is no constancy of M =10 and the concentration is changing, the time variation of the concentration of element is analyzed (c10), so that the detailed examination (c12) is needed when the abnormalities in the concentration of element (c11) is diagnosed. Especially, in the case of calcium element, when the values of M of the hair root part and the tip part of hair are in the vicinity of 100, and when the value of M of the tip part is about 10 and that of the hair root is 10 to 100, Ca agent (Ca ingredient is 900 to 1200mg) must be taken every day for about ten days. As a result, in the case that the value of M does not come back to the normal value, the detailed examination becomes necessary. The illness that appears by continuation of the condition of M =100 is called calcium paradox disease such as diabetes mellitus and Alzheimer disease, and the detailed examination is required. As described later, in the process that breast cancer develops, the state that the value of M is 100 continues for a long time, and the value decreases slowly to the normal value 10 in about one year. Therefore, in the case of that the tip part shows 100 and the hair root shows the intermediate value between 10 and 100, the points between them are analyzed with interval of 1-2cm, and the early discovery of breast cancer is made by investigating of the variation behavior of M. Besides breast cancer, for instance, large intestine cancer is also a calcium paradox disease, and since it is expected that the similar variation progress in M is seen, the detailed examination is also carried out.

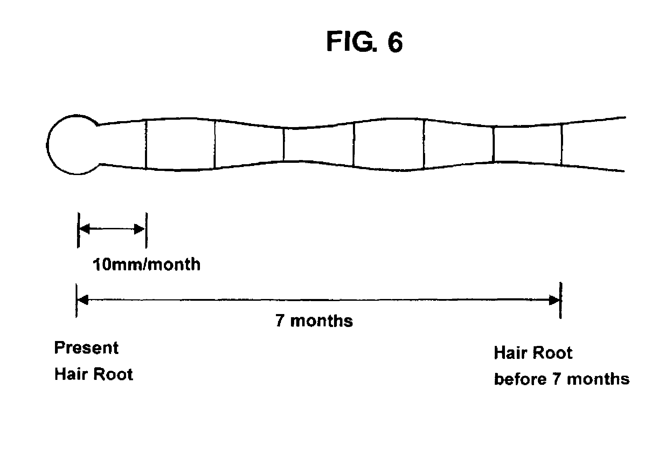

FIG. 6 is the enlarged model diagram of one hair. Since the hair grows about 10mm in length through one month, by measuring the contents of the element for two or more arbitrary parts, it is possible to diagnose the sick omen and sick progress from the property of its time variation. As shown in the method for evaluating physical conditions described in FIG. 5, if the values of M for two or more arbitrary parts are obtained, the time variation of the content of element can be derived from the time progress, and this is the important characteristic of the present invention.

Here, the relation between blood serum and hair is stated in detail. Many kinds of protein such as albumin, globulin and fibrinogen are contained in the blood serum. The concentration of calcium $[Ca]_S$ in blood serum is maintained at 10 mg/dL. The one half is included in protein (mainly albumin), the protein has the concentration of 4 to 5 g/dL, and the protein of 1 g contains calcium of 1 mg. The remaining one half exists as calcium ion $Ca^{2+}$. That is, the total calcium concentration $[Ca]_S$ in blood serum is the sum of the ion concentration $[Ca]_l$ and the concentration $[Ca]_P$ included in protein phase, and it is expressed by $[Ca]_S = [Ca]_l + [Ca]_P$.

Since calcium ion plays the role of signal transmission, the calcium ion concentration $[Ca]_l$ is controlled to be fixed strictly in any cases, namely it is kept constant as $[Ca^{2+}] = [Ca]_l$. On the other hand, $[Ca]_P$ varies to some extent. According to the knowledge of calcium paradox, generally, the cell has calcium pumps and calcium ion channels in the cell membrane. The pumps are always working in order to pump Ca out of the cell, and in calcium enough, the $Ca^{2+}$ channels are closed, so that the intracellular calcium ion concentration is maintained at almost zero.

On the other hand, hair is made by the hair matrix cell in the hair follicle surrounded by blood vessels, and is growing with a rate of about 0.3mm per day. In the cell, generally, there are storage sources of calcium such as endoplasmic reticulum and mitochondria. However, in steady-state growth of hair, the concentration of element in hair must balance with supply from blood independently of the internal calcium sources of the hair matrix cell. Here, the calcium concentration in hair is considered phenomenalogically.

In calcium enough, the $Ca^{2+}$ channels are closed and only the protein carrying one half of the calcium concentration $[Ca]_S$ in the blood serum is taken into the hair matrix cell. Even if the process in which hair is grown up is complicated, in the steady-state growth, the calcium concentration in hair $[Ca]_{HE}$ is in agreement with supply from the blood serum. Namely, it is proportional to the calcium concentration $[Ca]_P$ in the protein phase of the blood serum. The formula (1) holds using a proportional constant k.

$$[Ca]_{HE} = k \, [Ca]_P \quad (1)$$

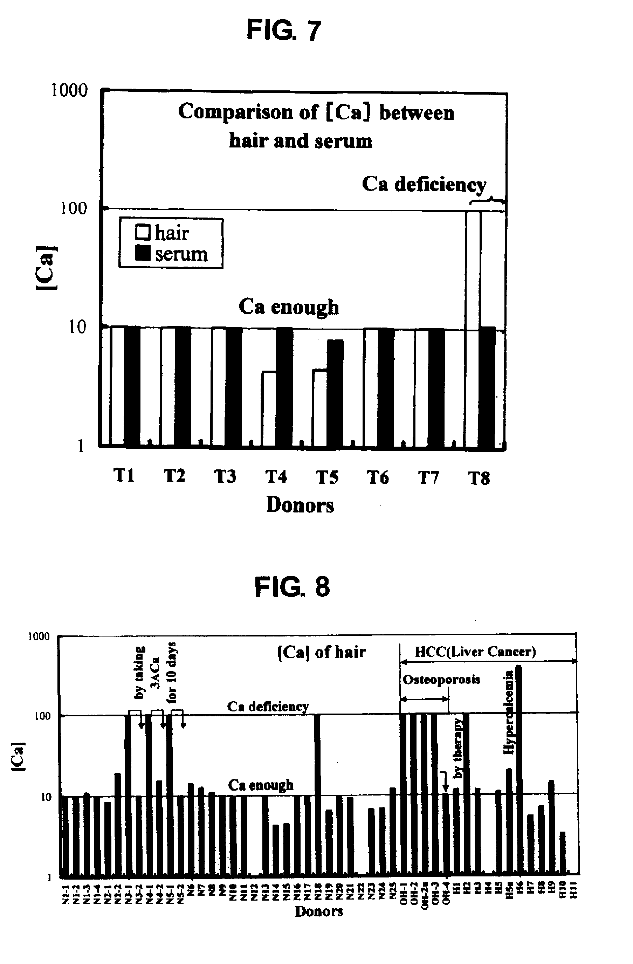

Since $[Ca]_P$ is one half of $[Ca]_I$, it is in agreement with the result $[Ca]_{HE} \propto [Ca]_I$ of FIG. 7 as mentioned later.

In the case of Ca deficiency, the parathyroid hormone PTH makes the $Ca^{2+}$ ion channels open. Although protein is taken into as mentioned above, Ca in the protein phase dissolves into the liquid phase in the hair matrix cell, and is also supplied from the liquid phase, so that the Ca in the protein is in equilibrium with the ion concentration in the blood serum through the ion channels. That is, $[Ca^{2+}] = [Ca]_I$ holds. Therefore, $[Ca]_I$ determines the calcium atom concentration $[Ca]_{PC}$ in the protein phase of the hair matrix cell. Then, the equilibrium relation between $[Ca]_I$ and $[Ca]_{PC}$ is considered.

In the case of calcium deficiency, it is shown by FIG. 7 and FIG. 8 as mentioned later that the hair calcium concentration $[Ca]_{HD}$ is proportional to the square of $[Ca]_S$. This suggests that Ca atoms are incorporated into the protein phase with forming pairs of Ca atoms.

The reaction rate that makes the pair of Ca atoms is proportional to the collision probability of calcium ion $Ca^{2+}$, and the dissociation rate of Ca atom in the protein phase is proportional to the concentration $[Ca]_{PC}$. Since these two rates become equal in chemical equilibrium, it can be written as $r \, [Ca]_{PC} = q \, [Ca]_I^2$ using the proportional constants r and q. Namely, it is written as $[Ca]_{HD} = k \, [Ca]_{PC} = k(q/r) \, [Ca]_I^2$, and is in agreement with the experimental result of $[Ca]_{HD} = [Ca]_{HE}^2$ by assuming

that q/r=1. The relation q/r=1 means that the chemical potential of calcium atom contained in the protein phase of the hair matrix cell is equal to that of $Ca^{2+}$ in the blood serum, and the formula (2) holds as follows.

$$[Ca]_{HD} = k \, [Ca]_I^2 \quad (2)$$

The formula (2) holds with q/r=1. Since it is $[Ca]_P \sim [Ca]_I$ in formula (1) and (2), $[Ca]_{HD} = [Ca]_{HE}^2$ is obtained, and this relation is in agreement with the result of FIGS. 7 and 8 as mentioned later.

In the case of calcium deficiency, since the calcium concentration in the protein phase of the hair matrix cell that determines $[Ca]_{HD}$ is in equilibrium with the constant $[Ca^{2+}]$ in the blood serum through the calcium channels, $[Ca]_{HD}$ becomes to be constant value as shown in FIG. 8 described later.

Since the average concentration of PTH in the blood serum increases gradually with age, it has been considered so far that the deficiency of calcium progresses with aging. However, it is shown from the result of hair analysis that calcium control works normally if the old man is also healthy, and the number of persons with calcium deficiency increases with aging. As shown in FIG. 8 explained later, although the number of persons is small, Ca deficiency occurs with the ratio of one person per five persons when the age is beyond 50 years, and occurs with the ratio of one person per 20 persons when the age is twenties.

<Detection of breast cancer>

For the early detection of breast cancer, calcified lesions (precipitates of calcium salts) are observed by X-ray mamography. The calcium source for the calcification is considered to be by the bone-resorbing activity with parathyroid hormone PTH. Unexpectedly, it is known that the calcitonin concentration in the blood serum is increased by breast cancer. This suggests that the Ca flow into the cancer site is increased by suppression of Ca overflow into cells at the whole-body scale.

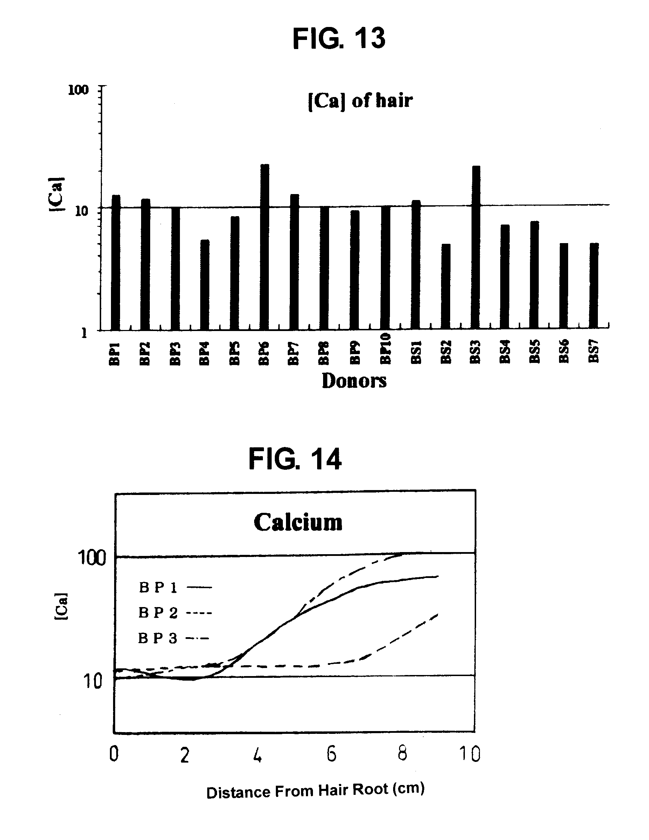

As a result of analyzing the hair root from 17 breast cancer patients (metastasis to bone for 7 out of 17), as shown in FIG. 13 mentioned later, all the hair roots show the calcium concentration of normal value (low level). As mentioned above, although the abnormal value due to calcium deficiency is seen for the ratio of one to five persons when the age is beyond 50 years, the result of "all the hair roots have the normal values with breast cancer" is unnatural.

Then, when the calcium concentration is measured from the hair root towards the tip for ten patients without metastasis to bone, as shown in FIG. 14 mentioned later, the calcium concentration gradually increases and the hair of all the patients shows the high concentration of abnormal value at distances of 7 to 10cm from the hair root. Beforehand, it is confirmed that the calcium concentration shows the normal value from root to tip for hair from persons with healthy steady-state life.

As seen from above results, the Ca deficiency continues over a long period before the calcification of breast cancer occurs, and the calcification is triggered with the calcium flood due to the calcium paradox. It can be concluded that the calcium concentration in hair decreases to the normal value with increase of calcitonin concentration in the blood serum.

Accordingly, by investigating the variation process of the calcium concentration of hair, the sign of breast cancer can be detected. Moreover, breast cancer may be prevented if the calcium deficiency disappears by supplements like 3ACa (Active Absorbable Algal Calcium). Women in the menopause are often in Ca deficiency, and it is considered that osteoporosis and breast cancer progress from the calcium deficiency. If she is affected by osteoporosis, Ca concentration of her hair has an abnormal value at the high level. While, breast cancer makes it to be the normal value at the low level. Therefore, it may be expected that those affected by osteoporosis do not develop breast cancer, and the woman with breast cancer does not suffer from osteoporosis.

In the following actual examples, the [Ca], for example, denotes the normalized concentration M of calcium, and M= 10 is the normal value.

[Example 1:Calcium Analysis]

FIG. 7 is the comparison diagram of the concentration of calcium contained in hair and blood serum of eight subjects. The subjects (Donors) are expressed with the axis of abscissa, and [Ca] (calcium concentration) is expressed with the axis of ordinate. The subjects of T1 to T8 are healthy persons, and in order to compare the analytical results of blood and hair, the hairs and bloods are extracted simultaneously.

Except in T5, the homeostasis is seen for the concentration $[Ca]_S$ of blood serum. The peak height of the concentration $[Ca]_H$ of hair is in agreement with $[Ca]_S$, except in T4, T5 and T8. That is, in the normal case, $[Ca]_H$ is proportional to $[Ca]_S$. In T8, although the blood serum has the normal $[Ca]_S$, the peak of $[Ca]_H$ of hair is very high. This high value of $[Ca]_H$ is due to the Ca shortage (deficiency), as seen from the experimental results for hairs of 37 subjects shown in FIG. 8 mentioned later.

The quantity of protein in the blood serum fluctuates, and when healthy, the density [PP] of protein phase is 4 to 5 g/dL. In the case that [PP] is large, since the whole quantity of calcium contained in protein is nearly constant, the number of Ca atoms per one protein molecule decreases. Since the number of protein molecules required to form the hair of 1 g is fixed, $[Ca]_{HE}$ becomes low if [PP] is large. This is the case for T4 of Fig. 7.

In T5, both of $[Ca]_S$ and $[Ca]_{HE}$ are lower than the normal values. This is because the quantity of Ca included in the protein phase becomes low due to the shift of pH of blood serum from the normal value. Since $[Ca]_I$ is always maintained at the normal value, the 20% decrease of $[Ca]_S$ results in the 40% decrease of $[Ca]_{HE}$ in hair incorporating only protein.

FIG. 8 is the comparison diagram of the concentration of calcium contained in hairs of 37 subjects. Donor is expressed with the axis of abscissa, and [Ca] (calcium concentration) is expressed with the axis of ordinate. Hair was extracted from 37 subjects. Among them, 11 persons are the patients of liver cancer (HCC) labeled H1 to H11. One subject is a patient with both of osteoporosis and liver cancer, and his hairs are four of OH-1, OH-2, OH-3 and OH-4, and the hairs were extracted with interval of four months, one month and five months. (OH-2u is for the part of 1 mm from hair root). The remaining subjects of 25 persons are healthy and labeled N1 to N25. Five subjects from N1 to N5 had taken the supplement of calcium for ten days, the hairs were extracted before and after the supplementation, and the hairs were encoded as N1-1 and N1-2, respectively. In total, the hair roots of total 37 persons were analyzed.

It was found from FIG. 8 that the high values of $[Ca]_H$ are due to the Ca deficiency. The normalized value of $[Ca]_H$ for most subjects distributes near the level of 10. The hair samples of signs N3, N4, N5, N18, OH and H2 show the peaks of $[Ca]_H$ with a height of 100. And the heights of peaks are the same. Namely, these peaks can be superimposed precisely. Then, the subjects of signs N3, N4, and N5 took supplement 3ACa (Active Absorbable Algal Calcium) with ratio of calcium 900mg every day for ten days. As a result due to extracting hairs and analyzing them again, $[Ca]_H$ decreased to the low normal values, as shown in FIG. 8

Moreover, the subject labeled OH has suffered from osteoporosis, so that the hair samples from OH-1 to OH-3 extracted over one year showed the high value of $[Ca]_H$, but $[Ca]_H$ decreased to normal value by therapy of cancer and intake of 3ACa supplement. It is found from these results that the high level of $[Ca]_H$=100 corresponds to Ca deficiency. It is considered that the high Ca concentration in the hair of H6 is due to hypercalcemia caused by PTHrP secreted from tumor.

The above results are summarized as follows. The Ca deficiency can be detected easily by X-ray fluorescence analysis of hair, and It becomes clear that (1) in Ca enough, the calcium concentration of hair shows the low value proportional to $[Ca]_S$ of blood serum and (2) in Ca deficiency, $[Ca]_H$ increases to the high level proportional to the square of $[Ca]_S$.

[Example 2: Iron Analysis]

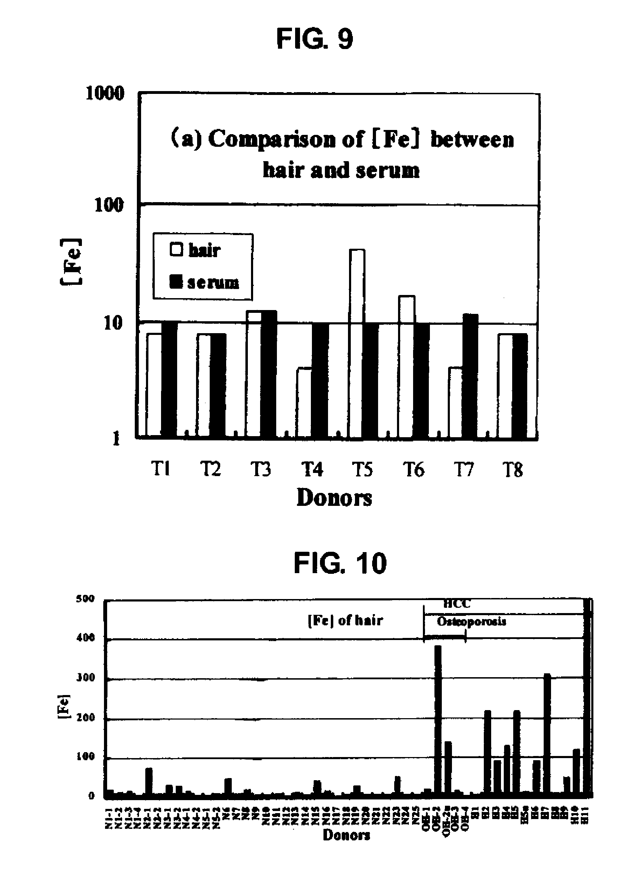

FIG. 9 is the comparison diagram of the concentration of iron contained in hair and blood serum of eight healthy persons. Subject (Donor) is expressed with the axis of abscissa, and [Fe] (iron concentration) is expressed with the axis of ordinate. T4 corresponds to the case that the density [PP] of protein phase in blood serum is large as has been described for T4 of FIG. 7. This means that Fe is also brought into hair by protein in the same manner of Ca. It is found from FIG. 9 that the same phenomenon as $[Ca]_H$ occurs.

FIG. 10 is the comparison diagram of the concentration of iron contained in hair of 37 subjects. Subject (Donor) is expressed with the axis of abscissa, and [Fe] (iron concentration) is expressed with the axis of ordinate. It is seen from FIG. 10 that ten or more abnormal persons in iron concentration exist even in 25 healthy persons. It is known that an active oxygen (toxicity, carcinogen) is produced in a body when much iron is taken in, so that it is considered that high $[Fe]_H$ means generation of said active oxygen. Moreover, it is found that there are many persons having abnormal iron concentration in liver-cancer patients (hepatocellular carcinoma, HCC).

[Example 3: Copper Analysis]

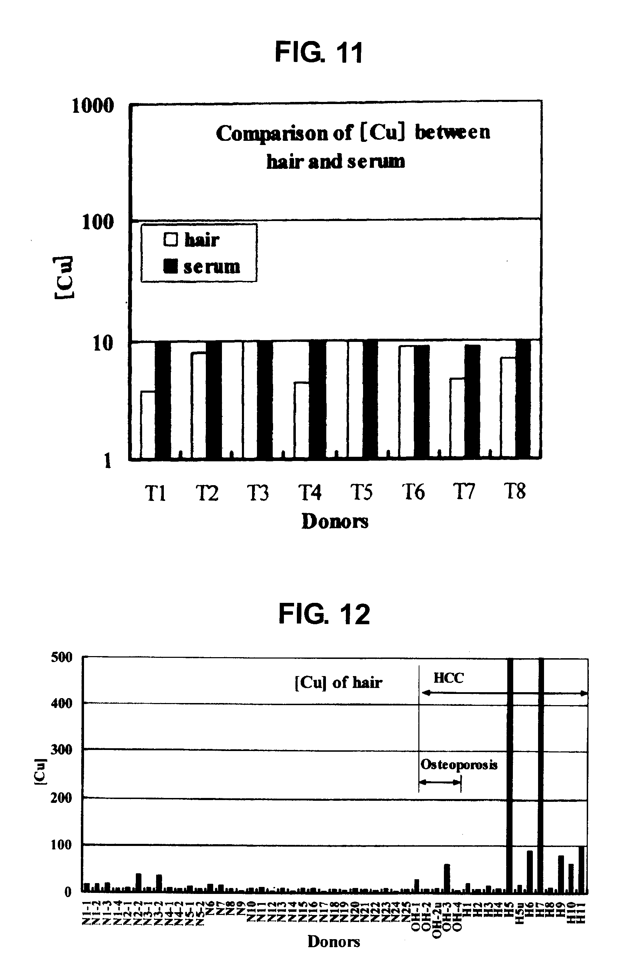

FIG. 11 is the comparison diagram of the concentration of copper contained in hair and blood serum of eight healthy persons. Subject (Donor) is expressed with the axis of abscissa, and [Cu] (copper concentration) is expressed with the axis of ordinate. T4 corresponds to the case that the density [PP] of protein phase in blood serum is large as has been described for T4 of FIG. 7. This means that Cu is also brought into hair by protein in the same manner of Ca.

FIG. 12 is the comparison diagram of the concentration of copper contained in hair of 37 subjects. Donors (Subjects) are expressed with the axis of abscissa, and [Cu] (copper concentration) is expressed with the axis of ordinate. It is found from FIG. 12 that there are abnormal persons of high copper concentration in liver-cancer patients.

[Example 4: Breast Cancer]

FIG. 13 is the comparison diagram of the concentration of calcium contained in hairs of 17 breast-cancer patients. Patients (Donors) are expressed with the axis of abscissa, and [Ca] (calcium concentration) is expressed with the axis of ordinate. Hairs were extracted from 17 patients of breast cancer. Among them, BP1 to BP10 are the primary cancer, and the remaining BS1 to BS7 are the hairs from the patients with metastasis to bone.

As illustrated in FIG. 13, all 17 patients of breast cancer showed the calcium concentration (low level) of normal values. Then, for the patients without metastasis to bone, the calcium concentration was measured towards the tip from the hair root.

FIG. 14 is the hysteresis curve diagram of the concentration of calcium contained in shafts form root to tip for 3 patients of breast cancer. The distance (cm) from the hair root is expressed with the axis of abscissa, and [Ca] (calcium concentration) is expressed with the axis of ordinate. Calcium concentration was measured towards the tip from the hair root of hair for three breast cancer patients BP1, BP2, and BP3 without metastasis to bone. As illustrated in FIG. 14, calcium concentration increases slowly and all hair from the patients shows the high concentration to be the abnormal value at distances of 7 to 10cm apart from the hair root. Additionally, in the hair of person that continues the healthy and steady state, it is confirmed that the calcium concentration shows the normal value from the hair root to the tip. It is possible to diagnose the generation of breast cancer by use of the hysteresis curve diagram of calcium concentration, and it can be used for diagnosis of its omen and progress.

It was proved from examples 1, 2, 3 and 4 that the method for evaluating physical conditions of the present invention can easily detect the variations of the content of element, especially the calcium deficiency, and can prevent various illnesses caused from the calcium deficiency. Moreover, for a trace element such as Fe and Cu as well as Ca, the exact diagnosis can be attained in comparison with the concentration of element of other specimens and the past concentration of element in the same person.

Although concentration is useful as the content of element in the present invention and not only [logP-logS] but also the normalized concentration (M or logM) defined by [logP-logS]/[logP-logS]st=logM is the best for said concentration, the other concentrations and normalization approaches are also useful. All the concentrations that can perform the mutual comparison between subject and healthy person can be used for said concentration, for example the concentration per unit volume or unit weight can be adopted. It is needless to say that not only the fluorescent X-ray intensity but also the other physical quantity such as element mass, element weight, element mol number, the number of atom, electric resistance, dielectric constant and magnetism are applicable for said concentration of element. In the same manner, it is needless to say that the present invention is not limited to the above-described embodiments; and various modifications and design changes, etc. are included in the scope of the present invention within this limits which do not deviate from the technical spirit of the present invention.

Industrial Applicability

Since the method for evaluating physical conditions of the present invention can measure correctly the concentration of element contained in head hair or body hair and can compare with other concentration of element or the past concentration of element, it is widely applicable in the field of not only medicine but also gene engineering and biochemistry.

**Claims**

**1.** A method for evaluating physical conditions **charac-**

terized in that content of an element contained in arbitrary point of head hair or body hair is measured and the physical conditions are evaluated from said content of the element.

2. The method for evaluating physical conditions according to claim 1, wherein said contents of the element contained in two or more points of said head hair or body hair are measured, and a time change of said contents of the element is derived by comparing said contents of the element.

3. The method for evaluating physical conditions according to claim 1 or 2, wherein said evaluation of the physical conditions is performed by using of said one or more head hairs or body hairs extracted periodically.

4. The method for evaluating physical conditions according to claim 1, 2 or 3, wherein said evaluation of the physical conditions is performed by comparing said contents of the element of said head hair or body hair with the content of the element contained in other specimen aside from hair of the same person.

5. The method for evaluating physical conditions according to claim 1, 2, 3 or 4, wherein said content of the element contained in said head hair or body hair of a subject is compared with content of the element contained in head hair or body hair of a healthy person, and said physical conditions are evaluated to be abnormal when said content of the element of said subject is larger or smaller than that of said healthy person.

6. The method for evaluating physical conditions according to any of claims 1 to 5, wherein said content of the element is a concentration of the element that is calculated from said content of the element at said measurement point of said head hair or body hair.

7. The method for evaluating physical conditions according to claim 6, wherein an excitation beam is irradiated to said measurement point of said head hair or body hair, a fluorescent X-rays emitted from said measurement point is measured, and said concentration of the element is derived from said fluorescent X-ray intensity.

8. The method for evaluating physical conditions according to claim 7, wherein said fluorescent X-ray intensity (P) is expressed by the decimal logarithm (logP) and said concentration of the element is given by the value (logP-logS) obtained by deducting the background value (logS) independent of the kind of elements from said decimal logarithm value (logP).

9. The method for evaluating physical conditions according to claim 8, wherein said concentration of the element for said subject is written as subject concentration (logP-logS), said concentration of the element for said healthy person is written as healthy person concentration (logP-logS)st, a normalized concentration M is defined by the formula logM = (logP - logS)/(logP - logS)st, and said evaluation for the physical conditions is performed by said normalized concentration M.

10. The method for evaluating physical conditions according to any of claims 1 to 9, wherein said element is a trace element such as Ca, Fe, Cu, Zn, Se, Sr and Rb.

11. The method for evaluating physical conditions according to any of claims 7 to 10, wherein said excitation beam is synchrotron radiation.

12. The method for evaluating physical conditions according to any of claims 1 to 11, wherein said evaluation is a diagnosis of breast cancer.

13. The method for evaluating physical conditions according to any of claims 1 to 11, wherein said evaluation is performed together with medical detailed examination.

# FIG. 1

# FIG. 2

# FIG. 3

```
┌─────────────────────────┐
│   Extract of Subject Hair │────── a1
└─────────────────────────┘
            │
            ▼
┌─────────────────────────┐
│  Irradiation of Excitation│
│  Beam to Arbitrary Part   │────── a2
│       of Hair             │
└─────────────────────────┘
            │
            ▼
┌──────────────────────────────┐
│ Measurement of Fluorescent X-rays │── a3
└──────────────────────────────┘
            │
            ▼
┌────────────────────────────────────┐
│ Logarithm Expression:Fluorescent X-rays │── a4
└────────────────────────────────────┘
            │
            ▼
┌────────────────────────────┐
│   Measurement of Peak (logP) │
│ Specified Element(Ca,Fe,Cu,Zn··) │── a5
└────────────────────────────┘
            │
            ▼
┌────────────────────────────┐
│  [logP-logS]／[logP-logS]st  │────── a6
│         =logM               │
└────────────────────────────┘
            │
            ▼
```

$$[logP-logS]／[logP-logS]st =logM$$

a7 — ◇ M : about 10 ◇ ── Yes ──▶ [ Normal ] ── a8

No

a9 — ◇ M≫10 or M≪10 ◇ ── No ──▶ [ Attention ] ── a10

                                ▼

                        [ Detailed Exam. for Need ] ── a11

Yes ▼

[ Abnormality of Concentration ] ── a12

     ▼

[ Detailed Exam. ] ── a13

# FIG. 4

```
┌─────────────────────────┐
│  Extract of Subject Hair │ ─────── b1
└─────────────────────────┘
            │
            ▼
┌─────────────────────────┐
│ Irradiation of Excitation│
│ Beam to Arbitrary Part   │ ─────── b2
│        of Hair           │
└─────────────────────────┘
            │
            ▼
┌───────────────────────────────┐
│ Measurement of Fluorescent X-rays│ ─────── b3
└───────────────────────────────┘
            │
            ▼
┌──────────────────────────────────┐
│ Logarithm Expression:Fluorescent X-rays │ ─────── b4
└──────────────────────────────────┘
            │
            ▼
┌──────────────────────────────┐
│ Measurement of Peak (logP) : Ca │ ─────── b5
└──────────────────────────────┘
            │
            ▼
┌──────────────────────────────┐
│ [logP−logS]／[logP−logS]st     │ ─────── b6
│        =logM                  │
└──────────────────────────────┘
            │
            ▼
```

$$[logP-logS]\diagup[logP-logS]st = logM$$

b7 — ◇ Hair Root, Tip M:about 10 ──Yes──▶ ☐ Normal ─────── b8

No

b9 — ◇ Hair Root, Tip M:about 100 ──Yes──▶ ☐ Ca Supplementation ─────── b10
▼ Hair Root Analysis ─────── b11

No

b12 — ◇ Root M:10~100 Tip M:about10 ──Yes──▶ ☐ Ca Supplementation ─────── b13
▼ Hair Root Analysis ─────── b14

No

☐ Abnormality ─────── b15

▼

☐ Detailed Exam. ─────── b16

# FIG. 5

Extract of Subject Hair — c1

Irradiation of Excitation Beam to Two or More Parts — c2

Measurement of Fluorescent X-Ray Spectrum of Measured Points — c3

Logarithm Expression Fluorescent X-rays — c4

Measurement of Peak (logP) Specified Element(Ca,Fe,Cu,Zn··) — c5

for Measured Points [logP-logS]／[logP-logS]st =logM — c6

Hair Growth Rate: 1cm/month Concentration Hysteresis M vs.Time(Length) — c7

c8 — M:about 10(Const)

Yes

Normal — c9

No

Time Variation Analysis:M — c10

Concentration Abnormality — c11

Detailed Exam. — c12

# FIG. 6

10mm/month

7 months

Present
Hair Root

Hair Root
before 7 months

# FIG. 7

# FIG. 8

# FIG. 9

(a) Comparison of [Fe] between hair and serum

# FIG. 10

## FIG. 11

Comparison of [Cu] between hair and serum

□ hair
■ serum

[Cu] vs Donors (T1–T8)

## FIG. 12

[Cu] of hair

HCC

Osteoporosis

Donors

## FIG. 13

[Ca] of hair

## FIG. 14

# FIG. 15

(15A)                              (15B)

Normal Value

Ageing
Calcium Shortage

# FIG. 16

# FIG. 17

<table>
<tr><td colspan="2" align="center">**INTERNATIONAL SEARCH REPORT**</td><td>International application No.<br>PCT/JP2005/014627</td></tr>
</table>

A. CLASSIFICATION OF SUBJECT MATTER
*G01N33/50* (2006.01), *G01N23/223* (2006.01), *G01N33/483* (2006.01)

According to International Patent Classification (IPC) or to both national classification and IPC

B. FIELDS SEARCHED

Minimum documentation searched (classification system followed by classification symbols)
*G01N33/50* (2006.01), *G01N23/223* (2006.01), *G01N33/483* (2006.01)

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X | JP 2004-45133 A (Hyogo Science and Technology Association),<br>12 February, 2004 (12.02.04),<br>Par. No. [0020]<br>(Family: none) | 1-13 |

☐ Further documents are listed in the continuation of Box C.   ☐ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search<br>29 November, 2005 (29.11.05) | Date of mailing of the international search report<br>13 December, 2005 (13.12.05) |
|---|---|
| Name and mailing address of the ISA/<br>Japanese Patent Office | Authorized officer |
| Facsimile No. | Telephone No. |

Form PCT/ISA/210 (second sheet) (April 2005)

**EP 1 777 518 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004045133 A